(19) Europäisches Patentamt
European Patent Office
Office européen des brevets

(11) EP 2 927 650 A1

(12) EUROPEAN PATENT APPLICATION

(43) Date of publication:
07.10.2015 Bulletin 2015/41

(51) Int Cl.:
$G01F\ 1/74^{(2006.01)}$          $G01F\ 25/00^{(2006.01)}$

(21) Application number: 14290101.6

(22) Date of filing: 04.04.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicants:
• Services Pétroliers Schlumberger
75007 Paris (FR)
Designated Contracting States:
FR
• Schlumberger Holdings Limited
Road Town, Tortola 1110 (VG)
Designated Contracting States:
GB NL
• Schlumberger Technology B.V.
2514 JG The Hague (NL)
Designated Contracting States:
AL AT BE BG CH CY CZ DE DK EE ES FI GR HR HU IE IS IT LI LT LU LV MC MK MT NO PL PT RO RS SE SI SK SM TR

(72) Inventors:
• Roux, Gilles
92140 Clamart (FR)
• Mauborgne, Marie-Laure
92140 Clamart (FR)
• Saenger, Richard
92140 Clamart (FR)
• Luling, Martin
92140 Clamart (FR)

(74) Representative: Rzaniak, Martin
Etudes & Productions Schlumberger
1, rue Henri Becquerel, BP 202
92142 Clamart Cedex (FR)

(54) Fluid analysis using electron-positron annihilation

(57)    A method for analyzing fluids using electron-positron annihilation is provided. In one embodiment, the method includes emitting positrons from a radioactive source of a multiphase flow meter and detecting photons generated from annihilation of electrons and the positrons. Detecting the photons can include detecting a first subset of the photons transmitted through a measurement conduit of the multiphase flow meter and detecting a second subset of the photons transmitted through a reference conduit of the multiphase flow meter. The detection of the first and second subsets of photons can then be used in determining a characteristic of a fluid within the measurement conduit. Additional systems, devices, and methods are also disclosed.

FIG. 5

EP 2 927 650 A1

## Description

## BACKGROUND

### Field

This disclosure relates to flow measurement devices, and more particularly, to multiphase flow meters.

### Description of the Related Art

Wells are generally drilled into subsurface rocks to access fluids, such as hydrocarbons, stored in subterranean formations. The subterranean fluids can be produced from these wells through known techniques. Operators may want to know certain characteristics of produced fluids to facilitate efficient and economic exploration and production. For example, operators may want to know flow rates of produced fluids. These produced fluids, are often multiphase fluids (e.g., those having some combination of water, oil, and gas), making measurement of the flow rates more complex.

Various systems can be used to determine flow rates for multiphase fluids. In some systems, multiphase fluids are separated into their constituent phases and these phases are then individually tested to determine flow rates. Other systems include multiphase flow meters that can be used to measure flow rates of multiphase fluids without separation. These multiphase flow meters may be smaller and lighter than traditional separators and test units, and the ability to measure flow rates without separation may be desirable in some instances. Both the traditional separator systems and the multiphase flow meter systems can also be used, separately or together, to determine other fluid characteristics of interest.

### SUMMARY

Certain aspects of some embodiments disclosed herein are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

In one embodiment of the present disclosure, a method for analyzing a fluid includes emitting positrons from a radioactive source of a multiphase flow meter and detecting photons generated from annihilation of electrons and the positrons. Detecting these photons can include detecting a first subset of the photons transmitted through a measurement conduit of the multiphase flow meter and a second subset of the photons transmitted through a reference conduit of the multiphase flow meter. The method also includes using the detection of the first and second subsets of photons in determining a characteristic of a fluid within the measurement conduit.

In another embodiment of the present disclosure, an apparatus includes a radioactive source material, with first and second conduits positioned on opposite sides of the radioactive source material. A first detector is positioned on an opposite side of the first conduit from the radioactive source material, and a second detector is positioned on an opposite side of the second conduit from the radioactive source material. The apparatus also includes a controller that can compare gamma radiation received at the first detector and gamma radiation received at the second detector to count occurrences of coincident gamma-ray photons received by the first and second detectors.

In an additional embodiment, a fluid analysis method is provided. This fluid analysis method includes operating a multiphase flow meter to detect coincident gamma-ray photons generated through electron-positron annihilation. The gas fraction of a fluid within a conduit of the multiphase flow meter can be determined based on the detected coincident photons.

Various refinements of the features noted above may exist in relation to various aspects of the present embodiments. Further features may also be incorporated in these various aspects as well. These refinements and additional features may exist individually or in any combination. For instance, various features discussed below in relation to the illustrated embodiments may be incorporated into any of the above-described aspects of the present disclosure alone or in any combination. Again, the brief summary presented above is intended just to familiarize the reader with certain aspects and contexts of some embodiments without limitation to the claimed subject matter.

## BRIEF DESCRIPTION OF THE DRAWINGS

So that the manner in which the above recited features can be understood in detail, a more particular understanding may be had when the following detailed description is read with reference to certain embodiments, some of which are illustrated in the appended drawings in which like characters represent like parts throughout the drawings. It is to be noted, however, that the appended drawings illustrate only some embodiments and are therefore not to be considered limiting of its scope, and may admit to other equally effective embodiments.

FIG. 1 generally depicts components of an apparatus, such as a multiphase flow meter, for analyzing a fluid in accordance with one embodiment of the present disclosure.

FIG. 2 is a block diagram of components of a computer of the apparatus of FIG. 1 in accordance with one embodiment.

FIGS. 3 and 4 depict an emitter and a detector of radiation positioned about a fluid conduit to enable irradiation of fluid within the conduit and measurement of radiation transmitted through the fluid in accordance with one embodiment.

FIG. 5 depicts certain components of an apparatus for analyzing fluid, the components including a radioactive source and detectors positioned along two fluid conduits to enable detection of radiation transmitted through each fluid conduit in accordance with one embodiment.

FIG. 6 is another example of the components of FIG. 5, with the fluid conduits being provided with Venturi throats in accordance with one embodiment.

FIG. 7 is a graph of a photon spectrum for a radioactive source depicting various features, including a peak corresponding to gamma radiation resulting from electron-positron annihilation, in accordance with one embodiment.

FIG. 8 generally illustrates the use of foil with the radioactive source of FIG. 5 to generate additional radiation that can be transmitted through the fluid conduits and received by the detectors in accordance with one embodiment.

FIG. 9 is a flow chart for using electron-positron annihilation to determine a characteristic of an analyzed fluid in accordance with one embodiment.

FIG. 10 is a flow chart for determining characteristics of a fluid based on coincident photon detection in accordance with one embodiment.

## DETAILED DESCRIPTION

It is to be understood that the present disclosure provides many different embodiments, or examples, for implementing different features of various embodiments. Specific examples of components and arrangements are described below for purposes of explanation and to simplify the present disclosure. These are, of course, merely examples and are not intended to be limiting.

When introducing elements of various embodiments, the articles "a," "an," "the," and "said" are intended to mean that there are one or more of the elements. The terms "comprising," "including," and "having" are intended to be inclusive and mean that there may be additional elements other than the listed elements. Moreover, any use of "top," "bottom," "above," "below," other directional terms, and variations of these terms is made for convenience, but does not mandate any particular orientation of the components.

Embodiments of the present disclosure generally relate to fluid analysis using radiation. More particularly, in at least some embodiments, a fluid analysis apparatus (e.g., a multiphase flow meter) includes a measurement conduit and a reference conduit. Radiation transmitted through fluids in these conduits is detected and used to determine characteristics of the fluids. Any suitable radiation could be used, such as electromagnetic or nuclear radiation. In some instances, detected electromagnetic radiation includes photon pairs emitted via electron-positron annihilation and coincident detection of the photons of these pairs is used in determining fluid characteristics of interest.

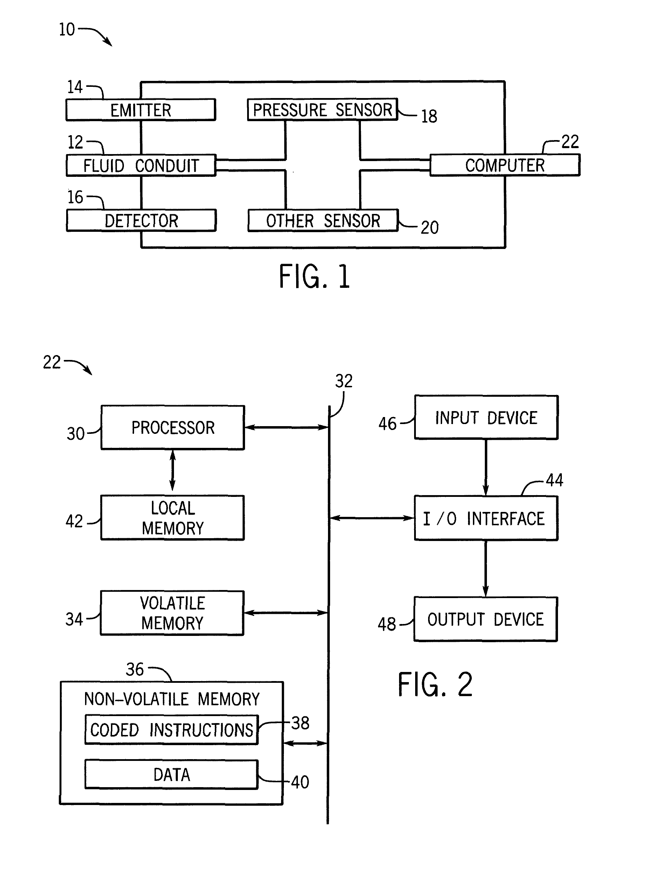

Turning now to the drawings, an apparatus 10 for analyzing fluid is generally depicted in FIG. 1 in accordance with one embodiment. While certain elements of the apparatus 10 are depicted in this figure and generally discussed below, it will be appreciated that the apparatus 10 may include other components in addition to, or in place of, those presently illustrated and discussed. Moreover, while the apparatus 10 may be provided in the form of a flow meter (e.g., a multiphase flow meter) as described below in connection with certain embodiments, the apparatus 10 could be provided in other forms as well. Further, in at least some instances the apparatus 10 is used to analyze fluids drawn from subterranean formations. Such analysis could be performed on fluids by the apparatus 10 (e.g., a multiphase flow meter) downhole (within a well) or at the surface.

As depicted, the apparatus includes a fluid conduit 12 for receiving a fluid. The apparatus 10 further includes an emitter 14 of radiation. In at least some embodiments, the emitter 14 can emit electromagnetic radiation directly or indirectly, such as in the form of emitted beta radiation that interacts with other particles to produce electromagnetic radiation. One example of this indirect emission of electromagnetic radiation by the emitter 14 is the emission of positrons that collide with electrons to produce gamma radiation. The apparatus 10 also includes a detector 16 of electromagnetic radiation (which in some instances can also or instead detect other radiation), a pressure sensor 18 (e.g., one or both of a pressure transmitter and a differential-pressure transmitter), and one or more additional sensors 20 (e.g., a temperature sensor). To facilitate certain measurements, such as flow rate, the fluid conduit 12 can have a tapered bore (e.g., a Venturi throat) to constrict fluid flow. Further, in at least one embodiment the emitter 14 and detector 16 are positioned about a Venturi throat in the fluid conduit 12 such that the detector 16 receives radiation that has been transmitted through fluid within the Venturi throat. As described in greater detail below, the apparatus 10 can include multiple fluid conduits 12 and detectors 16 to enable detection of gamma-ray photons emitted through electron-positron annihilation and transmitted through different fluid conduits to facilitate determination of desired fluid characteristics.

The apparatus 10 includes a computer 22 (which may also be variously referred to as a controller or a control unit) for determining characteristics of fluid within the fluid conduit 12. In at least some embodiments, the computer 22 is provided in the form of a flow computer coupled with the other depicted components in a single unit to facilitate installation of a flow meter in a larger system (e.g., an oilfield apparatus). More specifically, the computer 22 is operable to determine characteristics of fluids within the fluid conduit 12 from measurements collected by the other components. For example, the computer 22 can determine pressure and flow rate of the fluid. Further, a computer 22 of a multiphase flow meter can determine attenuation by the fluid of various levels of radiation by comparing the amount of radiation emitted from the emitter 14 to the portion of such radiation actually received by the detector 16. The computer 22 can also use this information to calculate phase fractions (e.g., proportions of oil, gas, and water) for a multiphase fluid within the fluid conduit 12. Single-phase flow rates can be achieved by combining the phase fraction measurements together with the total flow rate measurement. And as described below, in at least some embodiments the computer 22 uses a coincident counting technique for photons resulting from electron-positron annihilation to facilitate the determination of certain fluid characteristics, such as the gas fraction.

The computer 22 can be a processor-based system, an example of which is provided in FIG. 2. In this depicted embodiment, the computer 22 includes at least one processor 30 connected by a bus 32 to volatile memory 34 (e.g., random-access memory) and non-volatile memory 36 (e.g., flash memory and a read-only memory (ROM)). Coded application instructions 38 and data 40 are stored in the non-volatile memory 34. For example, the application instructions 38 can be stored in a ROM and the data 40 can be stored in a flash memory. The instructions 38 and the data 40 may be also be loaded into the volatile memory 34 (or in a local memory 42 of the processor) as desired, such as to reduce latency and increase operating efficiency of the computer 22. The coded application instructions 38 can be provided as software that may be executed by the processor 30 to enable various functionalities described herein. Non-limiting examples of these functionalities include determination of incident photon count rates on a detector, counting of coincident detections of photons by multiple detectors, and calculation of attenuation rates and phase fractions for a fluid. In at least some embodiments, the application instructions 38 are encoded in a non-transitory computer readable storage medium, such as the volatile memory 34, the non-volatile memory 36, the local memory 42, or a portable storage device (e.g., a flash drive or a compact disc).

An interface 44 of the computer 22 enables communication between the processor 30 and various input devices 46 and output devices 48. The interface 44 can include any suitable device that enables such communication, such as a modem or a serial port. In some embodiments, the input devices 46 include one or more sensing components of the apparatus 10 (e.g., detectors 16, pressure sensors 18, other sensors 20) and the output devices 48 include displays, printers, and storage devices that allow output of data received or generated by the computer 22. Input devices 46 and output devices 48 may be provided as part of the computer 22 or may be separately provided.

Further, while the computer 22 could be located with the fluid conduit 12 and sensing components of the apparatus 10 as a unitary system (e.g., a flow meter), the computer 22 could also be located remote from the other components. Further, the computer 22 could be provided as a distributed system with a portion of the computer 22 located with the sensing components at the fluid conduit 12 and the remaining portion of the computer 22 located remote from the fluid conduit 12.

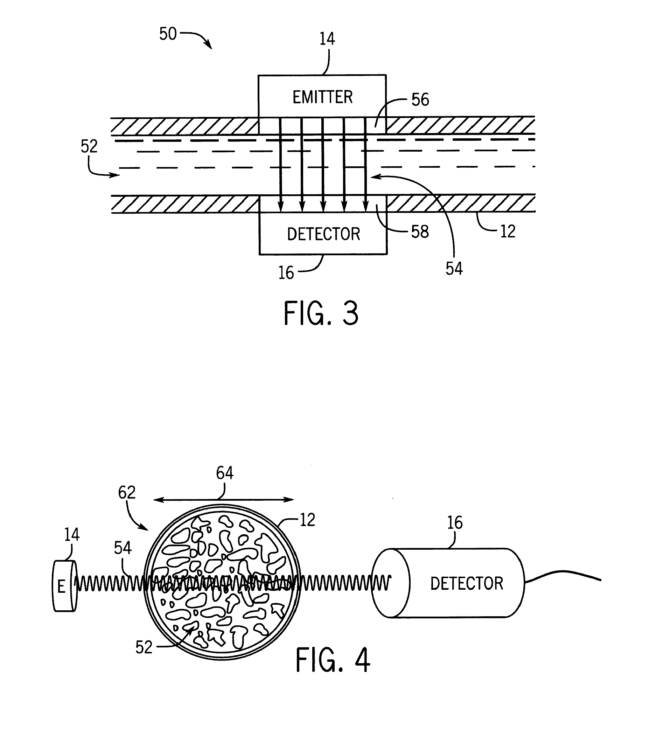

Additional details regarding operation of an emitter 14 and a detector 16 with a single conduit 12 may be better understood with reference to FIGS. 3 and 4. The emitter 14 and the detector 16, which may also be referred to as components of a spectrometer or densitometer 50, are arranged about the fluid conduit 12 in any suitable manner that allows the detector 16 to receive radiation (e.g., electromagnetic radiation) transmitted through fluid within the fluid conduit 12 from the emitter 14 (either directly or indirectly). As presently shown, the emitter 14 and the detector 16 are coupled opposite one another about the fluid conduit 12. Fluid 52 within the fluid conduit 12 is irradiated with electromagnetic or other radiation 54. Some of the radiation 54 may be absorbed or scattered by the fluid 52, but a portion of the radiation 54 is received by the detector 16. Windows 56 and 58 isolate the emitter 14 and the detector 16 from the fluid 52, while still permitting the radiation 54 to be transmitted from the emitter 14 and received by the detector 16. Particularly, the windows 56 and 58 are at least partially transparent to radiation to be emitted from the emitter 14 and read by the detector 16.

The emitter 14 can produce electromagnetic radiation of any suitable frequency and energy within the electromagnetic spectrum. In some embodiments the emitter 14 includes one or more radioactive sources that emit gamma rays and other nuclear radiation. More specifically, in at least some embodiments the emitter 14 includes a radioactive source, such as Sodium-22, that undergoes beta decay and emits positrons that collide with electrons to create pairs of gamma-ray photons, which can be considered to be emitted, indirectly, from the radioactive source.

As generally shown in FIG. 4, the emitter 14 and the detector 16 can be positioned on opposite sides of a Venturi throat 62 in the fluid conduit 12. This arrangement allows measurement of the linear attenuation coefficient, $\lambda_m$ (E), of the fluid 52 for electromagnetic radiation at a given energy E according to the Beer-Lambert law:

$$\lambda_m(E) = \frac{1}{d}\ln\left(N_0(E)/N(E)\right),$$

in which d is the throat diameter 64, $N(E)$ is the amount of transmitted photons (the quantity of photons detected by the detector 16), and $N_0(E)$ is the empty pipe count rates (the quantity of photons generated directly or indirectly by the emitter 14 that would reach the detector 16 but for interference by a medium, such as the fluid 52, in the throat 62).

In some applications, a flow meter can be used to determine the flow speed, $u$, of a fluid at the wellhead during production. Using the Bernoulli effect, the flow meter can measure the pressure drop $\Delta p$ in a pipe section with a reduced diameter (e.g., Venturi throat diameter 64) compared to the full-diameter pipe and relate it to the kinetic energy density, $E_{kin}$:

$$\Delta p \sim E_{kin} = \frac{1}{2}\rho u^2$$

The bulk fluid density, $\square$, can be measured separately to allow determination of the speed, $u$. In some flow meters, this density measurement uses the attenuation of photons (gamma-rays) by Compton scattering of the electrons in a medium (e.g., fluid 52) between a radiation source (e.g., emitter 14) and detector (e.g., detector 16). The radiation source can include a relatively weak source, such as Barium-133, and the detector can include a scintillation crystal with a photomultiplier tube. The source can emit photons at several energy levels and the relative attenuation of these photons allows determination of the gas and liquid fractions in multiphase flow. The mixture density can be computed from the gas and liquid fractions using reference densities of gas and liquids, as recomputed at the line pressure and temperature.

In such flow meters, the measurement accuracy is limited by the statistical error from the counts in the detector and is linked to the activity of the source. Low count rates (e.g., due to a weak source) increase the uncertainty in the measurements. The gas fraction measurement accuracy and mixture density computation errors at very high gas volume fractions (e.g., above ninety percent) are governed by the count rate statistics, which become increasingly relevant with higher gas fractions (count rate N $\pm\sqrt{N}$), as well as by the gas reference density computation from standard to line conditions and gas composition, which may differ from a reference calibration (thereby introducing further uncertainty).

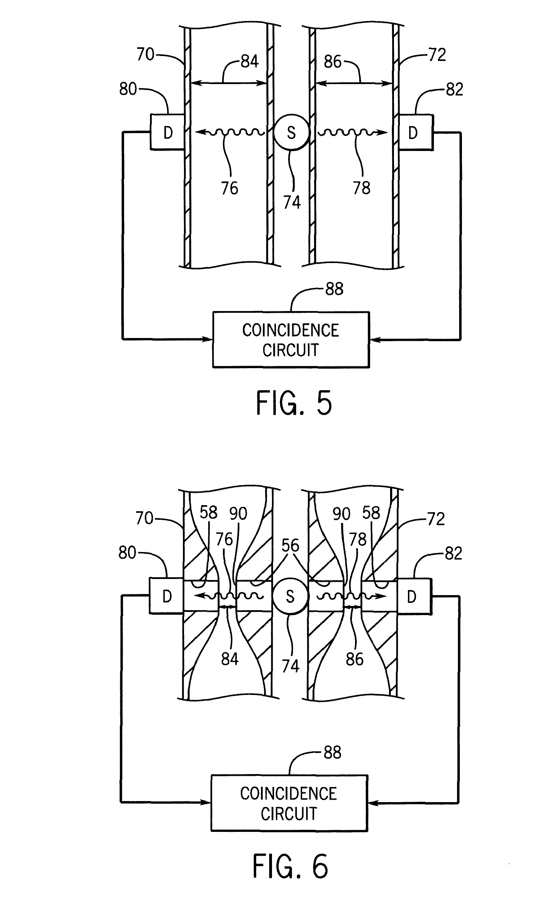

In at least some embodiments of the present disclosure, however, the gamma-ray transmission measurement through a single conduit described above is replaced or supplemented with a two-photon coincidence measurement technique using multiple fluid conduits. An example of components of an apparatus for enabling such measurement is generally illustrated in FIG. 5. As depicted, these components include fluid conduits 70 and 72, a radioactive source 74, and detectors 80 and 82. It will be appreciated that the components depicted in FIG. 5 can be part of the multiphase flow meter or other flow analysis apparatus 10 described above. The fluid conduit 12 can be embodied by the separate fluid conduits 70 and 72, the detector 16 can be embodied by the detectors 80 and 82, and the emitter 14 can be embodied by the radioactive source 74, for example. Further, it is noted that the fluid conduits 70 and 72 are simplified in FIG. 5 for explanatory purposes and can have other features and configurations. For instance, as shown in FIG. 6, the fluid conduits 70 and 72 can have Venturi throats 90 and windows 56 and 58 to facilitate measurements and analysis of fluids within the conduits. In other embodiments, the fluid conduit 70 could be provided with a Venturi throat 90 and the fluid conduit 72 could be provided without a Venturi throat (e.g., provided as a cylindrical pipe having an inner diameter equal to that of the Venturi throat 90 of the fluid conduit 72).

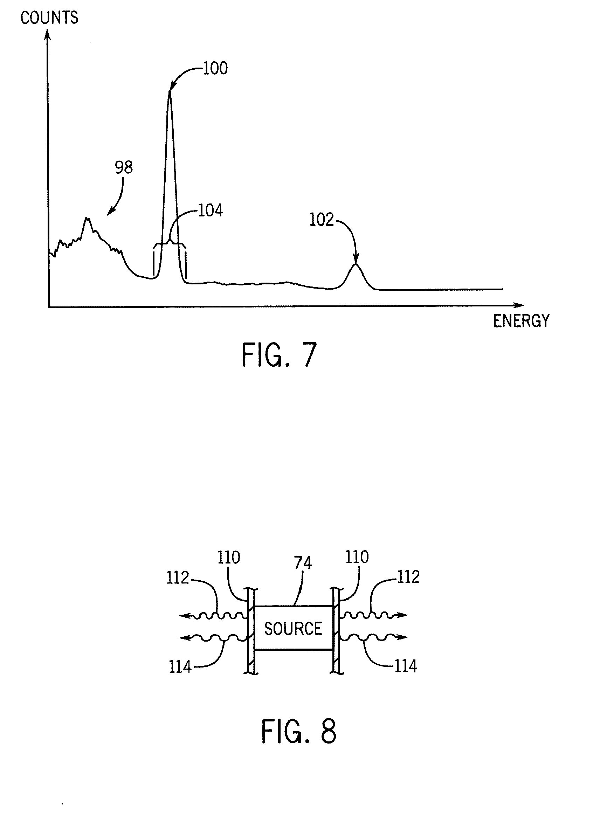

The radioactive source 74 can include any suitable material, and could be provided as a sealed radioactive source contained within a housing that provides at least some radiation shielding. In at least some embodiments, the radioactive source 74 includes a $\beta^+$-radioactive source material, such as Sodium-22, that emits positrons through beta decay. These positrons collide with nearby electrons, ultimately resulting in annihilation of the positrons and the electrons in pairs. Each electron-positron annihilation event creates two gamma-ray photons of identical energy (e.g., 511 keV) that are emitted from the annihilation site in opposite directions, as generally represented by photons 76 and 78. By way of example, a photon spectrum for a Sodium-22 source is generally depicted in FIG. 7 and includes a peak 100 (corresponding to 511 keV photons from electron-positron annihilation). This spectrum also includes counts for lower energy levels 98 and a peak 102 corresponding to gamma radiation emitted from the Sodium-22 source at 1.274 MeV.

Returning to FIGS. 5 and 6, the conduit 70 and the conduit 72 are arranged on opposite sides of the radioactive source 74. The detectors 80 and 82, which can be provided in any suitable form (e.g., with a scintillator crystal and photomultiplier tube), are positioned on opposite sides of the conduits 70 and 72 from the radioactive source 74. Further, the radioactive source 74 can be positioned in-line with the detectors 80 and 82, and also with any Venturi throats 90 (as in FIG. 6).

The flux of photons resulting from electron-positron annihilation can be monitored across the fluid conduits 70 and 72 with the detectors 80 and 82. A fluid of interest can be provided in the fluid conduit 70 (also referred to as a measurement conduit) and a reference fluid can be provided in the fluid conduit 72 (also referred to as a reference conduit). In some embodiments, the fluid of interest flows through the measurement conduit 70 while the reference conduit 72 is provided as a "dummy pipe" that serves as a reference chamber. Produced gas (e.g., dry gas) can be stored in this reference chamber, allowing the density of the produced gas to be measured. In at least some embodiments, a fluid mixture of gas and liquid is routed through the measurement conduit 70 while a dry gas is provided in the reference conduit 72. Further, the fluids in the conduits 70 and 72 could contain sand or other solid particulates that impact attenuation of radiation passing through the fluids.

The measurement paths through the measurement conduit 70 and the reference conduit 72 are functionally identical in at least some embodiments. That is, the conduits 70 and 72 have equal widths 84 and 86 at the measurement point between the source 74 and the detectors 80 and 82, the radioactive source 74 is positioned equidistant between the detectors 80 and 82, and the materials and dimensions of the fluid conduits 70 and 72 (including any windows 56 and 58) are substantially similar along the paths traveled by radiation (e.g., gamma photons 76 and 78) from the source 74 to the detectors 80 and 82 so that the impact of any differences on the photon counts measured with the detectors is negligible. The fluid conduits 70 and 72 could also have identical volumes in some embodiments. In the case of scintillator detectors 80 and 82, the detector crystal material and geometry can be selected to promote reliable capture and absorbance of high energy photons while reducing "escapes" from within the crystal. Additionally, the distance from the radioactive source 74 to each of the detectors 80 and 82 can vary between different embodiments, and in at least some instances the distance could be changed symmetrically within embodiments (each detector 80 and 82 could be moved closer to the source 74 by identical amounts) to compensate for decay of the source 74 over time.

In some embodiments, a time-amplitude coincidence technique is used to measure photons of similar energy (e.g., 511 keV for a Sodium-22 source) that are detected by the detectors 80 and 82 at nearly the same time. More specifically, a timing window (e.g., ten nanoseconds) can be used to account for small differences in the paths traveled by each photon of a photon pair generated through electron-positron annihilation. These small differences can be attributed to various factors, such as the stochastic natures of positron direction when emitted from the source 74 and positron location when annihilation occurs. As used herein, coincident detection includes detection of a pair of photons received by the detectors within the same time window and is not limited to simultaneous detection.

Further, although photons generated through electron-positron annihilation often begin with an energy level of 511 keV, some portion of this energy can be lost as the photons travel to the detectors. Additionally, two detectors could measure energies of the photons differently. To account for such variation, an energy measurement window covering a range of energy levels (e.g., 450 keV to 570 keV) can be used in the coincidence technique rather than a specific energy level (e.g., 511 keV). This allows two photons received by the two detectors to be registered as coincident so long as the two photons fall within the same timing window and have energies within the energy measurement window. One example of this energy measurement window is provided in FIG. 7 as window 104, which includes the peak 100 and has upper and lower boundaries set to the edges of the distribution associated with that peak 100.

A coincidence circuit 88, which may be provided as part of the computer 22 (FIG. 1), is provided for comparing gamma radiation received at the detector 80 to that received at the detector 82 to track (e.g., count occurrences of) coincident gamma-ray photons received by the two detectors, such as in the manner described above. The coincidence circuit 88 can include a comparator that determines whether incident photons on the detectors 80 and 82 are coincident (within the same timing window and energy measurement window) and a counter that is incremented with each positive identification of coincidence. Although the coincidence circuit 88 could be implemented as separate hardware, the coincidence circuit 88 could be implemented via programming, such as with the circuit 88 embodied by the processor 30 executing coded instructions 38 for performing this function.

Assuming the gamma-ray detection instrumentation and the paths traveled by the photons to the detectors 80 and 82 were well-balanced and the same fluid (e.g., dry gas) was in both the conduits 70 and 72, the count rates of photons detected by the respective detectors 80 and 82, correlated in time, would be nearly equal (allowing for stochastic variation). Further assuming negligible attenuation in the case of dry gas, the coincident count rate (i.e., the number of occurrences of coincident photon pairs being received by the detectors 80 and 82) would be substantially equal (allowing for stochastic variation and the negligible attenuation) to the count rates of the detectors 80 and 82. The ratio of the coincident count rate to the count rate of the detector 82 would then also be substantially equal to one.

In practice, fluids of interest routed through (or otherwise provided within) the measurement conduit 70 can have liquids that absorb or scatter photons emitted by the source 74, causing the coincident count rate to decrease proportionally with the proportion of liquids in the conduit 70. The gas fraction (GF) of a fluid mixture within the measurement conduit 70 can be determined based on the ratio of the coincident count rate to the count rate of the detector 82 (for the reference conduit 72). Further, the photon count rates of the detector 82 (the count of those photons transmitted through fluid, such as dry gas, in the reference conduit 72) can be used for normalization of photon count rates of the detector 80 to reduce the effects of gas density variations due to environmental conditions (e.g., pressure, temperature and composition).

The determined gas fraction (GF) of a fluid mixture of interest within the measurement conduit 70 can be used in high gas or wet gas instances to determine liquid fraction (LF) of the fluid and to compute the gas flow rate, $q_{Gas}$:

$$q_{Total} = K \cdot \sqrt{2 \cdot \frac{\Delta P_{Venturi}}{\rho_{Mixture}}}$$

$$q_{Gas} = q_{Total} \cdot \text{GF}$$

where $q_{Total}$ is the total flow rate, $\rho_{Mixture}$ is the density of the fluid mixture within the measurement conduit 70, $\Delta P_{Venturi}$ is the pressure drop in a Venturi tube, and $K$ is a discharge coefficient based on characteristics of the measurement conduit 70.

The density of the fluid mixture within the measurement conduit 70 can be determined from an empty pipe measurement and attenuation of radiation (e.g., 511 keV photons) by the fluid mixture. Similarly, the in-line gas density for the gas within the reference conduit 72 can be computed based on an empty pipe measurement and attenuation of gamma or other radiation by the gas in the conduit 72. It is noted that with no liquid fraction in the reference conduit 72, the density of a produced dry gas in the reference conduit 72 is stable, allowing the count rate of the detector 82 to be averaged over a long period of time and the uncertainty in the measurement to be reduced. This determined dry gas density can then be used with the measured fluid mixture density of measurement conduit 70 to determine the liquid fraction density of the fluid mixture.

The precision of the coincidence measurements described above will depend on various parameters, such as the accuracy of detectors 80 and 82 and the construction of the components used in the flow analysis apparatus 10. For example, materials with low absorption (low atomic numbers), such as beryllium or boron carbide ($B_4C$), may be used for components along the photon transmission paths from the source 74 to the detectors 80 and 82 to reduce interference with the photons.

In some embodiments, a metal foil can be added to the photon transmission paths from the source 74 to the detectors 80 and 82. For instance, as generally depicted in FIG. 8, metal foil 110 can be provided on opposite sides of the radioactive source 74. Interaction of higher-energy radiation 112 (e.g., gamma radiation from electron-positron annihilation) with the foil 110 can generate lower-energy radiation 114 (e.g., x-rays). The amount of attenuation of both the radiation 112 and the radiation 114 can be determined with the detectors 80 and 82. In the case of detector 80, which measures attenuation by a fluid mixture in the measurement conduit 70, the measurement of attenuation at multiple energy levels facilitates three-phase fraction measurement (e.g., oil, water, and gas). Although the foil 110 on the side of the source 74 having a reference conduit 72 could be omitted, the inclusion of foil 110 between the source 74 and each of the conduits 70 and 72 balances the photon transmission paths to the detectors 80 and 82 so as not to unequally impact the count rates of either detector. Any suitable foil 110 could be used, such as tungsten, cadmium, or another high density metal.

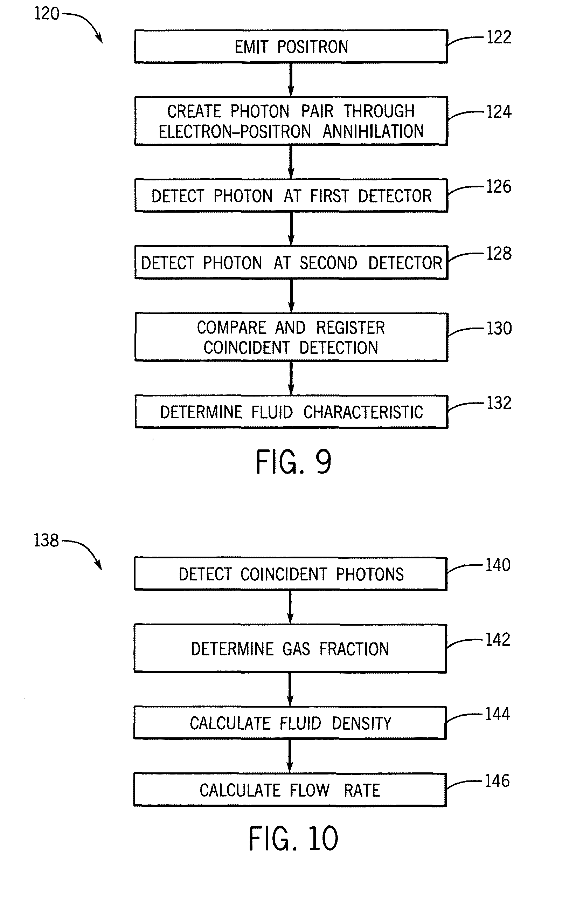

With the foregoing in mind, an example of a process for using electron-positron annihilation for analyzing a fluid of interest is generally represented by flow chart 120 in FIG. 9. In this embodiment, a positron is emitted (block 122) from a radioactive source, such as source 74 of a multiphase flow meter, and a gamma-ray photon pair is created through electron-positron annihilation (block 124). The photons of the pair are transmitted in opposite directions such that one photon is detected at a first detector (block 126) and the other photon is detected at the second detector (block 128). The detections of these photons by the two detectors (e.g., detectors 80 and 82) are compared and registered as a coincident detection (block 130). In some instances, the registration of the coincident detection can include incrementing a coincidence counter in response to determining that the detections of the two photons occurred near in time within a timing window.

Although described in the preceding paragraph in the context of the detection of one photon pair from electron-positron annihilation, it will be appreciated that in practice a large number of positrons will be emitted from the radioactive source and then be annihilated through interaction with electrons, leading to the creation of numerous pairs of gamma-ray photons. Many of these photons may not be detected by the detectors of the apparatus, due to attenuation of photons initially traveling toward the detectors and emission of the photons in other directions (not toward the detectors). But a first subset of the photons generated from electron-positron annihilation travel through one conduit (e.g., measurement conduit 70) to a first detector (e.g., detector 80) and a second subset of these photons travel through another conduit (e.g., reference conduit 72) to a second detector (e.g., detector 82). The detections of these photons by the detectors can be compared to identify and register coincident detections (e.g., by incrementing a coincidence counter).

The detection of these first and second subsets of annihilation-generated photons may be used in determining a characteristic of a fluid within one of the conduits (block 132), such as described above. For example, these first and second subsets of detected photons can be used to determine a characteristic (e.g., gas fraction, density) of a fluid mixture produced from a well and routed into the measurement conduit 70. In at least some instances, the determination of the fluid characteristic uses the number of coincident detections registered from the detectors.

An example of another process for analyzing a fluid of interest is generally represented by flow chart 138 in FIG. 10. In this embodiment, a multiphase flow meter or other fluid analysis apparatus is operated to detect coincident gamma-ray photons generated through electron-positron annihilation (block 140) and a gas fraction of a fluid within a conduit of the multiphase flow meter (or other fluid analysis apparatus) is determined based on the detected coincident photons (block 142), as described above. In some instances, such operation includes receiving one or more photon pairs generated through electron-positron annihilation, wherein one photon of each photon pair is received through the fluid within the conduit of the multiphase flow meter and another photon of the photon pair is received through an additional fluid within an additional conduit of the multiphase flow meter. The fluid in the conduit can be a liquid-gas mixture and the additional fluid in the additional conduit can be a dry gas. Fluid densities and flow rates can also be calculated (blocks 144 and 146). The density of a liquid-gas mixture in the conduit can be calculated based on attenuation of gamma radiation generated through electron-positron annihilation by the liquid-gas mixture within the conduit. The density of a dry gas within the additional conduit can also be calculated based on attenuation of gamma radiation generated through electron-positron annihilation by the dry gas. Additionally, the density of the liquid portion of the liquid-gas mixture within the conduit can be calculated based on the calculated density of the liquid-gas mixture within the conduit and the calculated density of the dry gas within the additional conduit.

Although the preceding description has been described herein with reference to particular means, materials and embodiments, it is not intended to be limited to the particulars disclosed herein; rather, it extends to all functionally equivalent structures, methods, and uses, such as are within the scope of the appended claims.

## Claims

1. A method comprising:

   emitting positrons from a radioactive source of a multiphase flow meter;
   detecting photons generated from annihilation of electrons and the positrons, wherein detecting the photons includes detecting a first subset of the photons transmitted through a measurement conduit of the multiphase flow meter and detecting a second subset of the photons transmitted through a reference conduit of the multiphase flow meter; and
   using the detection of the first and second subsets of photons in determining a characteristic of a fluid within the measurement conduit.

2. The method of claim 1, wherein determining the characteristic of the fluid within the measurement conduit includes determining a characteristic of a fluid mixture from a well.

3. The method of claim 2, wherein determining the characteristic of the fluid mixture from the well includes determining a gas fraction of the fluid mixture.

4. The method of claim 1, further comprising:

   providing a dry gas within the reference conduit.

5. The method of claim 1, further comprising:

   registering coincident detection of photons of the first subset transmitted through the measurement conduit and photons of the second subset transmitted through the reference conduit.

6. The method of claim 5, wherein using the detection of the first and second subsets of photons in determining the characteristic of the fluid within the measurement conduit includes using the registered coincident detection of the photons of the first and second subsets in determining the characteristic of the fluid within the measurement conduit.

7. The method of claim 5, wherein registering coincident detection of photons of the first and second subsets includes incrementing a coincidence counter in response to detecting that a photon of the first subset received by a first detector of the multiphase flow meter and a photon of the second subset received by a second detector of the multiphase flow meter are received within a coincidence timing window.

8. The method of claim 5, wherein registering coincident detection of photons of the first and second subsets includes registering coincident detection of photons of the first and second subsets having energies within an energy measurement window of the multiphase flow meter.

9. An apparatus comprising:

   a radioactive source material;
   first and second conduits positioned on opposite sides of the radioactive source material;
   a first detector positioned on an opposite side of the first conduit from the radioactive source material and a second detector positioned on an opposite side of the second conduit from the radioactive source material; and
   a controller operable to compare gamma radiation received at the first detector and gamma radiation received at the second detector to count occurrences of coincident gamma-ray

**EP 2 927 650 A1**

photons received by the first and second detectors.

10. The apparatus of claim 9, further comprising:

a multiphase flow meter having the radioactive source material, the first and second conduits, and the first and second detectors, and wherein the controller is a flow computer of the multiphase flow meter operable to calculate flow rates of fluids within the first conduit based on counted occurrences of coincident gamma-ray photons received by the first and second detectors.

11. The apparatus of claim 10, wherein the flow computer of the multiphase flow meter is operable to use photon count rates of the second detector for normalization of the photon count rates of the first detector to reduce effects of gas density variations due to environmental conditions.

12. The apparatus of claim 9, wherein at least one of the first or second conduits comprises a Venturi throat aligned with the radioactive source material.

13. The apparatus of claim 9, wherein foil is positioned between the radioactive source material and each of the first and second conduits to enable radiation passing through the foil to generate additional radiation of lower energy.

14. The apparatus of claim 9, wherein the radioactive source material comprises Sodium-22.

15. A method comprising:

operating a multiphase flow meter to detect coincident gamma-ray photons generated through electron-positron annihilation; and determining a gas fraction of a fluid within a conduit of the multiphase flow meter based on the detected coincident photons.

FIG. 1

FIG. 2

FIG. 3

FIG. 4

FIG. 5

FIG. 6

COUNTS

—100

98

—104

102—

ENERGY

## FIG. 7

110    74    110

112—        —112

SOURCE

114—        —114

## FIG. 8

120

| EMIT POSITRON | 122 |

↓

| CREATE PHOTON PAIR THROUGH ELECTRON–POSITRON ANNIHILATION | 124 |

↓

| DETECT PHOTON AT FIRST DETECTOR | 126 |

↓

| DETECT PHOTON AT SECOND DETECTOR | 128 |

↓

| COMPARE AND REGISTER COINCIDENT DETECTION | 130 |

↓

| DETERMINE FLUID CHARACTERISTIC | 132 |

## FIG. 9

138

| DETECT COINCIDENT PHOTONS | 140 |

↓

| DETERMINE GAS FRACTION | 142 |

↓

| CALCULATE FLUID DENSITY | 144 |

↓

| CALCULATE FLOW RATE | 146 |

## FIG. 10

**EP 2 927 650 A1**

Europäisches
Patentamt

European
Patent Office

Office européen
des brevets

# EUROPEAN SEARCH REPORT

Application Number

EP 14 29 0101

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/301899 A1 (DAVID PIERRE-YVES [FR]) 8 December 2011 (2011-12-08) * figure 1 * * paragraph [0140] * ----- | 9-14 | INV. G01F1/74 ADD. G01F25/00 |
| X | US 2013/313437 A1 (LI HONG DI [US] ET AL) 28 November 2013 (2013-11-28) | 15 | |
| Y | * figure 1 * * paragraphs [0001], [0002] * * paragraph [0031] * * first sentence; paragraph [0033] * * first sentence; paragraph [0035] * ----- | 1-8 | |
| Y | US 3 934 471 A (WHITE PERCY WILLIAM ET AL) 27 January 1976 (1976-01-27) * figure 7 * ----- | 1-8 | |

TECHNICAL FIELDS
SEARCHED        (IPC)

G01F
G01N

The present search report has been drawn up for all claims

| Place of search | Date of completion of the search | Examiner |
|---|---|---|
| The Hague | 1 October 2014 | Rambaud, Patrick |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................................

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 14 29 0101

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2014

| Patent document cited in search report | Publication date | Patent family member(s) | | Publication date |
|---|---|---|---|---|
| US 2011301899 A1 | 08-12-2011 | FR | 2939886 A1 | 18-06-2010 |
| | | US | 2011301899 A1 | 08-12-2011 |
| | | WO | 2010067038 A1 | 17-06-2010 |
| US 2013313437 A1 | 28-11-2013 | EP | 2662686 A1 | 13-11-2013 |
| | | US | 2013313437 A1 | 28-11-2013 |
| | | WO | 2012027945 A1 | 08-03-2012 |
| US 3934471 A | 27-01-1976 | GB | 1455021 A | 10-11-1976 |
| | | IN | 142338 A1 | 25-06-1977 |
| | | JP | S49113663 A | 30-10-1974 |
| | | US | 3934471 A | 27-01-1976 |

EPO FORM P0459

For more details about this annex : see Official Journal of the European Patent Office, No. 12/82